## Europäisches Patentamt
## European Patent Office
## Office européen des brevets

(11) Veröffentlichungsnummer: **0 242 511**
**B1**

(12) # EUROPÄISCHE PATENTSCHRIFT

(45) Veröffentlichungstag der Patentschrift:
13.06.90

(51) Int. Cl.⁵: **A61B 17/22**, G01S 15/89

(21) Anmeldenummer: 87101365.2

(22) Anmeldetag: 02.02.87

(54) Lithotripter.

(30) Priorität: 21.04.86 DE 3613457

(43) Veröffentlichungstag der Anmeldung:
28.10.87 Patentblatt 87/44

(45) Bekanntmachung des Hinweises auf die Patenterteilung:
13.06.90 Patentblatt 90/24

(84) Benannte Vertragsstaaten:
DE FR

(56) Entgegenhaltungen:
EP-A- 0 081 051
DE-A- 3 122 056

(73) Patentinhaber: Siemens Aktiengesellschaft,
Wittelsbacherplatz 2, D-8000 München 2(DE)

(72) Erfinder: Franke, Kurt, Dr., Lange Zelle 99,
D-8520 Erlangen(DE)

ACTORUM AG

## Beschreibung

Die Erfindung betrifft einen Lithotripter mit einem an einer Versorgungsschaltung angeschlossenen Stoßwellengenerator und mit einer Röntgeneinrichtung zur Darstellung eines zu zertrümmernden Steines, wobei die Röntgeneinrichtung ein dem Röntgenbild des zu zertrümmernden Steins entsprechendes elektrisches Ausgangssignal abgibt.

Die Röntgeneinrichtung kann dabei zur Ortung der zu zertrümmernden Steine benutzt werden. Dadurch ist es möglich, den Stoßwellengenerator genau auf den jeweils zu zertrümmernden Stein zu fokussieren. Damit das gesunde Gewebe außerhalb der zu zertrümmernden Steine bei der Stoßwellenbehandlung nicht in Mitleidenschaft gezogen wird, wird jeweils nur eine festgelegte Anzahl von Stoßwellen, z.B. 50 Stoßwellen, verabreicht. Die darauffolgende Einleitung einer neuen Stoßwelle oder einer neuen Stoßwellenserie muß von dem behandelnden Arzt neu ausgelöst werden. Es muß demgemäß nach einer Serie von z.B. 50 Stoßwellen ein Schalter zur Einleitung einer weiteren Serie von z.B. 50 Stoßwellen betätigt werden. Diese Maßnahme zwingt demgemäß zu einer besonderen Sorgfalt bei der Behandlung. Es ist jedoch möglich, daß z.B. nach dem Auslösen der zweiten Stoßwellenserie der Stein sich schon nach wenigen Stoßwellen aufgelöst hat. Die weiteren Stoßwellen wären dann nicht mehr erforderlich.

Der Erfindung liegt die Aufgabe zugrunde, einen Lithotripter der eingangs genannten Art so auszubilden, daß nur gerade so viele Stoßwellen verabreicht werden, wie dies zur Zerkleinerung des jeweils zu behandelnden Steins notwendig ist.

Diese Aufgabe ist erfindungsgemäß dadurch gelöst, daß der Röntgeneinrichtung Mittel zur Ermittlung der tatsächlichen Steingröße zugeordnet sind, denen das elektrische Ausgangssignal der Röntgeneinrichtung zugeführt ist, und daß bei Unterschreitung einer bestimmten Steingröße die Abschaltung der Versorgungsschaltung des Stoßwellengenerators erfolgt. Wenn also der Zerfall des zu zertrümmernden Steins bis zu einem vorgegebenen Wert fortgeschritten ist, wird die Behandlung mit Stoßwellen automatisch beendet. Dabei ist gemäß einer Variante der Erfindung vorgesehen, daß die Röntgeneinrichtung zwei Röntgensysteme aus je einem Röntgenstrahler und einem Strahlenempfänger aufweist, deren Zentralstrahlen in einer Ebene einen Winkel miteinander bilden, und daß die Mittel zur Ermittlung der tatsächlichen Steingröße einen Computer und für jedes Röntgensystem eine Detektionsschaltung aufweisen, wobei jeder Detektionsschaltung das elektrische Ausgangssignal des entsprechenden Röntgensystems zur Bildung eines der Steingröße entsprechenden elektrischen Signales zugeführt ist, und diese Signale dem Computer zur Berechnung der tatsächlichen Steingröße zugeführt sind, so daß der Computer bei Unterschreitung einer bestimmten Steingröße die Versorgungsschaltung des Stoßwellengenerators abschaltet. In diesem Falle läßt sich das der Steingröße entsprechende elektrische Signal aus den längs der Zeilen von mittels der Röntgensysteme gewonnenen Röntgenbilder des zu zertrümmernden Steins entsprechenden Fernsehsignalen auftretenden Spannungen gewinnen.

Ein Lithotripter mit einem an einer Versorgungsschaltung angeschlossenen Stoßwellengenerator und einer Röntgeneinrichtung zur Darstellung eines zu zertrümmernden Steins ist in der nicht vorveröffentlichten EP-A 0 211 680 beschrieben. Die Röntgeneinrichtung, die zwei Röntgensysteme aus je einem Röntgenstrahler und einem Strahlenempfänger aufweist, deren Zentralstrahlen in einer Ebene einen Winkel miteinander bilden, dient ausschließlich der Ortung des zu zertrümmernden Steins. Dabei geben die Röntgensysteme der Röntgeneinrichtung jeweils ein dem Röntgenbild des zu zertrümmernden Steins entsprechendes elektrisches Ausgangssignal ab, das dazu dient, die Röntgenbilder des zu zertrümmernden Steins auf Fernsehmonitoren darzustellen. Maßnahmen zur Ermittlung der tatsächlichen Steingröße und zur selbsttätigen Beendigung der Behandlung bei Unterschreitung einer bestimmten Steingröße sind nicht getroffen.

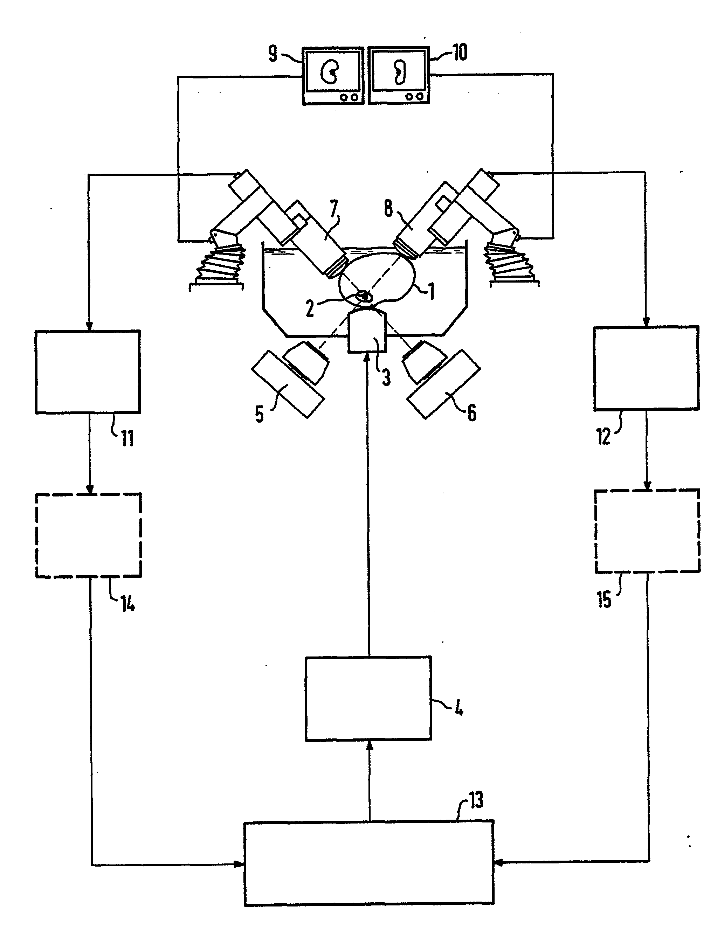

Die Erfindung ist nachfolgend anhand eines in der Zeichnung dargestellten Ausführungsbeispieles näher erläutert.

In der Zeichnung ist ein Patient 1 im Querschnitt dargestellt, der einen Nierenstein 2 aufweist, der durch die von einem Stoßwellengenerator 3 erzeugten Stoßwellen zertrümmert werden soll. Der Patient 2 liegt in einer mit Wasser gefüllten Wanne. Dem Stoßwellengenerator 3 ist eine Versorgungsschaltung 4 zugeordnet. Zur bildlichen Darstellung des Steins ist eine Röntgeneinrichtung mit zwei Röntgensystemen vorgesehen, bei der jedes Röntgensystem aus einem Röntgenstrahler 5, 6 und einem Röntgenbildverstärker 7, 8 mit nachgeschalteter Fernsehkette besteht. Die Zentralstrahlen der Röntgensysteme 5 bis 8 liegen in einer Ebene und kreuzen sich unter einem Winkel von z.B. etwa 90°. Die beiden so erzeugten Röntgenbilder werden auf zwei Monitoren 9, 10 dargestellt.

Die Fernsehsignale der beiden Röntgensysteme 5, 7 und 6, 8 werden zwei Detektionsschaltungen 11, 12 zugeführt, die die Größe des jeweiligen Steines in der jeweiligen Projektion ermitteln und ein entsprechendes elektrisches Signal einem Computer 13 zuführen. Der Computer 13 bestimmt aus den Größen in den beiden Röntgenbildern die tatsächliche Steingröße (Größe der bei der Zertrümmerung gebildeten Steinteilchen) und bewirkt die Abschaltung des Stoßwellengenerators 3, wenn die tatsächliche Steingröße einen vorgegebenen Wert unterschreitet.

Es ist auch möglich, jedem Kanal eine Vergleichsschaltung 14, 15 zuzuordnen, die für die Steingröße im jeweiligen Röntgenbild einen bestimmten Wert vorgibt. Wird dieser Wert unterschritten, und zwar in beiden Kanälen, so erfolgt die Abschaltung des Stoßwellengenerators 3.

Maßgebend ist, daß die Steingröße, nämlich die Größe der bei der Zertrümmerung gebildeten Steinteilchen, einen Wert erreicht, bei dem ein natürlicher Steinabgang möglich ist.

## Patentansprüche

1. Lithotripter mit einem an einer Versorgungsschaltung (4) angeschlossenen Stoßwellengenerator (3) und einer Röntgeneinrichtung (5, 6, 7, 8) zur Darstellung eines zu zertrümmernden Steins, wobei die Röntgeneinrichtung (5, 6, 7, 8) ein dem Röntgenbild des zu zertrümmernden Steins entsprechendes elektrisches Ausgangssignal abgibt, wobei der Röntgeneinrichtung (5, 6, 7, 8) Mittel (11, 12, 13) zur Ermittlung der tatsächlichen Steingröße zugeordnet sind, denen das elektrische Ausgangssignal der Röntgeneinrichtung (5, 6, 7, 8) zugeführt ist, und wobei bei Unterschreitung einer bestimmten Steingröße die Abschaltung der Versorgungsschaltung (4) des Stoßwellengenerators (3) erfolgt.

2. Lithotripter nach Anspruch 1, dadurch gekennzeichnet, daß die Röntgeneinrichtung (5, 6, 7, 8) zwei Röntgensysteme aus je einem Röntgenstrahler (5, 6) und einem Strahlenempfänger (7, 8) aufweist, deren Zentralstrahlen in einer Ebene einen Winkel miteinander bilden, und daß die Mittel (11, 12, 13) zur Ermittlung der tatsächlichen Steingröße einen Computer (13) und für jedes Röntgensystem eine Detektionsschaltung (11, 12) aufweisen, wobei jeder Detektionsschaltung (11, 12) das elektrische Ausgangssignal des entsprechenden Röntgensystems zur Bildung eines der Steingröße entsprechenden elektrischen Signals zugeführt ist und diese Signale dem Computer (13) zur Berechnung der tatsächlichen Steingröße zugeführt sind, so daß der Computer (13) bei Unterschreitung einer bestimmten Steingröße die Versorgungsschaltung (4) des Stoßwellengenerators (3) abschaltet.

## Claims

1. A lithotriptor with a shock wave generator (3) attached to a supply circuit (4) and with an x-ray device (5, 6, 7, 8) for representing a calculus to be disintegrated, wherein the x-ray device (5, 6, 7, 8) emits an electric output signal corresponding to the x-ray image of the calculus to be disintegrated, and wherein allocated to the x-ray device (5, 6, 7, 8) there are means (11, 12, 13) for detecting the actual calculus size, to which means the electric output signal of the x-ray device (5, 6, 7, 8) is supplied, and wherein if a specific calculus size is not reached disconnection of the supply circuit (4) of the shock wave generator (3) occurs.

2. A lithotriptor according to claim 1, characterised in that the x-ray device (5, 6, 7, 8) has two x-ray systems comprising in each case an x-ray source (5, 6) and a ray receiver (7, 8), the central rays of which form together in one plane an angle and in that the means (11, 12, 13) for detecting the actual calculus size have a computer (13) and for each x-ray system a detection circuit (11, 12), wherein the electric output signal of the corresponding x-ray system for forming an electric signal corresponding to the size of calculus is supplied to each detection circuit (11, 12) and these signals are supplied to the computer (13) for calculating the actual calculus size, so that the computer (13) disconnects the supply circuit (4) of the shock wave generator (3) if a specific calculus size is not reached.

## Revendications

1. Lithotriteur comportant un générateur d'ondes de choc (3), raccordé à un circuit d'alimentation (4), et un dispositif radiologique (5, 6, 7, 8) destiné à former l'image d'un calcul devant être fragmenté, et dans lequel le dispositif radiologique (5, 6, 7, 8) délivre un signal de sortie correspondant à l'image radiographique du calcul à fragmenter et qu'au dispositif radiologique (5, 6, 7, 8) sont associés des moyens (11, 12, 13), qui servent à déterminer la taille effective du calcul et auxquels est appliqué le signal électrique de sortie du dispositif radiologique (5, 6, 7, 8), et dans lequel, lorsque la taille du calcul est inférieure à une taille prédéterminée, le circuit d'alimentation (4) du générateur d'ondes de choc (3) est débranché.

2. Lithotriteur suivant la revendication 1, caractérisé par le fait que le dispositif radiologique (5, 6, 7, 8) comporte deux systèmes radiologiques constitués chacun d'un émetteur de rayons X (5, 6) et d'un récepteur de rayonnement (7, 8), dont les rayons centraux forment entre eux un angle dans un plan, et que les moyens (11, 12, 13) servant à déterminer la taille effective du calcul comprennent un ordinateur (13), ainsi que, pour chaque système radiologique, un circuit de détection (11, 12), et qu'à chaque circuit de détection (11, 12) est appliqué le signal électrique de sortie du système radiologique correspondant pour la formation d'un signal électrique correspondant à la taille du calcul, lesquels signaux issus des circuits de détection (11, 12) sont envoyés à l'ordinateur (13) pour calculer la taille effective du calcul, la réalisation étant telle que, lorsque la taille du calcul à fragmenter est inférieure à une taille déterminée, l'ordinateur (13) débranche le circuit d'alimentation (4) du générateur d'ondes de choc (3).